# EUROPEAN PATENT SPECIFICATION

(11) **EP 3 732 300 B1**
(45) Date of publication and mention of the grant of the patent: **08.03.2023**
(21) Application number: 18893431.9
(22) Date of filing: 25.12.2018
(51) Int. Cl.: C12Q 1/04, C12N 1/20

(54) **A NOVEL MEDIUM FOR GROWTH AND ANTIBIOTIC SUSCEPTIBILITY TEST OF MYCOBACTERIA**
NEUARTIGES MEDIUM FÜR WACHSTUMS- UND ANTIBIOTIKAEMPFINDLICHKEITSTESTS VON MYKOBAKTERIEN
NOUVEAU MILIEU DE CULTURE ET TEST DE SENSIBILITÉ AUX ANTIBIOTIQUES DE MYCOBACTÉRIES

(30) Priority: 29.12.2017 TR 201722985
(43) Date of publication of application: 04.11.2020
(73) Proprietor: Akdeniz Universitesi, Antalya (TR)
(72) Inventor: COBAN, Ahmet Yilmaz, Konyaalti/Antalya (TR)
(74) Representative: Sevinç, Cenk
(86) International application number: PCT/TR2018/050884
(87) International publication number: WO 2019/132836

(56) References cited:
- WO-A1-2016/124863
- WO-A1-2016/124863
- FR-A1- 3 033 575
- US-A1- 2001 055 787
- Rajdev Sangita ET AL: "Use of blood agar along with Lowenstein-Jensen media for rapid isolation of Mycobacterium tuberculosis for early drug susceptibility testing :Sangita Rajdev, Aarohi Patel, Summaiya Mulla, Journal of The Academy of Clinical Microbiologists", , 14 November 2014 (2014-11-14), pages 1-4, XP055866773, Retrieved from the Internet: URL:https://www.jacmjournal.org/printartic le.asp?issn=0972-1282;year=2014;volume=16; issue=2;spage=100;epage=103;aulast=Rajdev [retrieved on 2021-11-29]
- PALANGE P. et al.: "Evaluation of Culture Media for Isolation of Mycobacterium Species from Human Clinical Specimens", Cureus, vol. 8, no. 8, 30 August 2016 (2016-08-30) , page e757, XP055634527,

## Description

### Technical Field

This invention relates in particular to a novel medium developed with the aim of growing *Mycobacterium tuberculosis* leading to tuberculosis cases from clinical samples and ensuring the determination of their drug resistance and also for use in biochemical tests for identification of mycobacteria.

### State of the art

Tuberculosis (phthisis) is one of the oldest health problems that still cannot be solved completely all over the world and is a chronic, granulomatous bacterial and contagious infection disease caused by infection of *M. tuberculosis* bacillus.

The main characteristics of bacteria of *Mycobacterium* genus are that they grow slowly, they have resistance to acid, and they have high content of mycolic acid (lipid) in their cell walls. Considering from clinical aspect, *M. tuberculosis* is the most essential member of the genus because of its disease infection potential and close association with the public health and is the main cause of tuberculosis encountered in humans today.

Diagnosis of tuberculosis can be determined by clinical signs and symptoms, and by assessment of skin test result and also by identifying *M. tuberculosis* via biochemical tests in the mycobacteria cultured in culture medium.

Because of the difficulties in diagnosis and treatment of tuberculosis, it becomes harder to keep the disease under control. Therefore, studies are still being conducted to develop novel media especially for effective treatment, early identification of the agent and drug resistance.

Direct microscopic examination and acid-fast bacteria (AFB) staining method are used as a fast and simple method having most common usage in clinical studies. However, by means of microscopic examination, bacillus of *M. tuberculosis* is encountered in 40-80% of pulmonary tuberculosis cases. Therefore, the presence of AFB in direct examination neither makes the diagnosis of tuberculosis certain and nor gives an accurate result. This agent is required to be grown in a medium.

Mycobacterium culture methods used are the most frequently applied methods for the diagnosis of tuberculosis with regard to the fact that they allow for obtaining the isolates needed for the identification processes to be conducted after growing the bacteria, and for the bacteria to show their viabilities, and for the diseases to be accurately treated by performing the drug susceptibility tests, and for obtaining the epidemiological data. However, disadvantage of these methods is that the diagnosis of bacteria requires some time since the growth period of bacteria takes a long time.

It is possible to divide mycobacterium culture methods into two as solid and liquid media. The World Health Organization also suggests the simultaneous use of a solid and a liquid medium for the culturing of mycobacteria.

The culture processes in solid media are time-consuming and laborious. Several weeks are needed for colonies to become visible.

Solid media is divided into two as egg and agar-based media:
> Egg based media: These are Löwenstein-Jensen (LJ), Petragnani and American Trudeau Society (ATS). The most commonly used one is the LJ medium. It takes 18-24 days for the colonies to become visible. Furthermore, due to its high egg-based protein content and possibility that the antibiotics can be inactivated during its solidification by heating it up to 80°C brings along disadvantage in susceptibility tests.
> Agar-based media: Middlebrook 7H10 and 7H11 agars are the most preferred ones. Oleic acid-albumin-dextrose-catalase (OADC) enricher should be added in it before use. Mycobacteria become visible 10-12 days after cultivation while their media is transparent, thence the time to detect positivity is shorter compared to the LJ medium. In the case of a small number of bacteria in the sample or presence of strong decontamination, the monitoring of specific colonies may take a long time of about 6 to 8 weeks. Furthermore, it is expensive due to the enrichment used and in the case of contamination, medium losses occur since the whole surface of the medium is often influenced.

### Liquid Media:

The detection of mycobacteria by the liquid cultures is performed in shorter time and in higher sensitivity. They contain Middlebrook 7H9 and Dubos Tween albumin fluids therein. Fast culture systems are grouped as manual, semi-automated or fully automated systems according to the fact that whether they are evaluated by device and computer or not. However, the requirement of additional device and equipment for the automated systems in which this medium is used also bring along a disadvantage in terms of costs.

Sensitivity of LJ medium in the isolation of mycobacteria from clinical samples is lower compared to the Middlebrook 7H10, 7H11 and its liquid form 7H9 medium (broth) because of the reasons such as longer growth time and late detection of colony formation.

Mycobacteria are handled after being primarily grouped according to their growth times, growth temperatures, colony morphology that they created on the medium and pigment characteristics and by the determination of appropriate biochemical tests.

### Biochemical tests used to identify mycobacteria:

Catalase test, arylsulfatase test, growth in Mac Conkey agar without crystal violet, iron uptake, niacin accumulation, nitrate reduction, pyrazineamidase test, sodium chloride tolerance test, inhibition by thiophene-2-carboxylic acid hydrazide, tellurite reduction, hydrolyze of Tween 80, and urease activity is among the biochemical tests used in the identification of mycobacteria.

Determination of the growth characteristics of mycobacteria and their identification by conventional biochemical methods can take approximately 3-6 weeks or sometimes even longer.

Susceptibility methods are direct method by which cultivation of clinical samples, comprising acid fast bacteria in which calculating the average number of bacteria in one milliliter of the sample, into the media in a drug and drug-free manner after homogenization-decontamination procedures, and indirect method wherein the cultivation into drug and drug-free media after preparing appropriate inoculum by isolating the acid-fast bacteria in pure culture state from the clinical samples.

Middlebrook 7H10 and 7H11 media enriched with oleic acid-albumin-dextrose-catalase (OADC) and egg-based LJ medium are used in the susceptibility tests of mycobacteria.

The drugs developed against *M. tuberculosis* in the mid-1950s have been widely used for many years. Although effective drugs against tuberculosis have been on the market for many years and it has been considered as a treatable disease, the main reason of the difficulty in treatments is the development of resistance to anti-TB drugs. Application of biochemical tests used for the diagnosis of tuberculosis make the process difficult in terms of both cost and preparation times due to their above-mentioned disadvantages.

In the state of the art, WO2016124863 relates to a method for the enrichment and selective culture of mycobacteria using a culture medium comprising a nutritive component that stimulates the development and growth of mycobacteria and as selective agents at least 9-chloro-9-(4'-diethylamino)phenyl-9,10-dihydro-10-phenylacridine hydrochloride (C-390) and an agent that can inhibit *Pseudomonas aeruginosa* bacteria. In one example this document describes a medium based on the Löwenstein-Jensen medium comprising: 30 g of potato starch, 3.6 g of L-asparagine, 2.4 g of monopotassium phosphate, 0.24 g magnesium sulfate, 0.6 g magnesium citrate, 1 1 eggs homogenate, 12 ml glycerol and 51.2 mg C-390.

In the state of the art, the patent application US6951733B2 relates to a novel agar-based medium for the isolation, subculturing, and indirect or direct drug susceptibility test of *M. tuberculosis.* This agar medium suitable for the growth of *M. tuberculosis* comprises 200 units/ml of polymyxin B, 50 microgram/ml carbenicillin, 10 microgram/ml amphotericin B and 20 microgram/ml trimethoprim lactate of antimicrobial agents. It is mentioned that the final volume of the resulting agar medium contains bovine serum at a concentration of about 8% to 12%. The agar used to prepare the said medium is selected from the group consisting of Middlebrook and Cohn 7H10 and Middlebrook and Cohn 7H11. In that invention, bovine serum of 8% to 12% of the final volume of agar medium was used instead of OACD as an enricher.

In the state of the art, the medium mentioned in the application "CA2807357A1" contains the substances Glycerol 5.0 mL, sodium caseinate 2.0 g, L-asparagine 0.1 g, sodium propionate 4.0 g, dipotassium phosphate 0.5 g, magnesium sulfate 0.1 g, iron sulfate 0.001 g, 1.0 L water (750 ml of Artificial Sea Water + 250 mL of demineralized water), agar powder 15.0 g, final pH (at 25°C) 7.4 to 7.8.

The media existing in the state of the art mentioned above, has disadvantages due to the fact that growth of the bacteria takes a long time and that the costs are high.

### Problems aimed to be solved by the invention

The aim of the invention is to ensure that *M. tuberculosis* leading to tuberculosis and other mycobacteria are grown in a short period of time and that colony formation is detected earlier.

Further aim of the invention is to ensure that the antibiotic susceptibility of *M. tuberculosis* isolates is determined by using the solid and liquid medium as defined by the present invention.

### Description of Invention

This invention relates particularly to a novel medium developed with the aim to use in biochemical tests for culturing *M. tuberculosis* leading to tuberculosis cases and other mycobacteria from clinical samples, for determining the drug resistance in *M*. *tuberculosis* isolates and for identifying the mycobacteria.

The invention provides a medium which increases growth rate of mycobacteria by using an enrichment of animal origin together with substances in the medium, shortens determination time of mycobacteria and used for antibiotic susceptibility tests of *M. tuberculosis,* characterized in that; a liquid form of the medium comprises 3-4 grams of L-asparagine, 2-3 grams of monopotassium phosphate, 0.2-0.3 grams of magnesium sulfate, 15-20 grams of peptone, 0.3-1 grams of magnesium citrate, 3-7 milliliters of glycerol, 900-950 milliliters of distilled water and inactive sheep serum and/or sheep plasma in the rate of 5-10% of final volume of resulting medium, and a solid form of agar medium comprises 15 grams of agar in addition to above mentioned substances.

The invention further provides preparation methods of the liquid medium form and of the solid agar medium form as defined in claims 2 and 3.

For the simultaneously use of a solid and a liquid medium, analogous medium substances are used and the medium which brings along cost efficient advantages by using serum/plasma of animal origin instead of OADC as enrichment agent, and which at the same time shortens visibility time of mycobacteria for about a week is achieved.

The unexpected technical effect in the subject matter product is that; the medium formed by use of enrichment of animal origin together with the substances on the medium has both provided advantage in terms of costs and reduced the growing time of the mycobacteria. By this measure, it has reduced the determination time thereby the detection time of tuberculosis bacteria which is one of the biggest problems encountered in the state of the art. Thus, intervening to tuberculosis disease caused by *M. tuberculosis* in a shorter period of time come into being.

Another problem is that the enrichment agent such as OADC used in existing media is expensive. This problem is also eliminated by using enricher of animal origin in the subject matter of the invention. Furthermore, it is advantageous that it is cost efficient during the diagnosis considering that the disease is observed particularly in developing countries.

Both solid and liquid medium is achieved during forming of the medium that allow for subject matter mycobacteria to be cultured and their antibiotic susceptibilities to be tested.

Solid and liquid media are the formulations that provide maximum growth of bacteria. By means of substances forming the solid and liquid medium and of usage amounts of said substances, inactivation of the antibiotics is inhibited. The usage amounts of subject matter medium substances and the amount of enrichment added are therefore essential. The amount of the enricher used to increase the growth rate of mycobacteria is also important.

The prepared solid media is agar-based media containing asparagine, monopotassium phosphate, magnesium sulfate, peptone, agar, magnesium citrate, glycerol and distilled water. The medium in question is clear/transparent and the colonies begin to appear 5 to 7 days after cultivation.

The compositions and the amounts of the medium obtained are shown in Table 1.

**Table 1: Table showing the compositions and the substance amounts of the medium**

| Sequence No. | Compositions | Amount of substances |
|---|---|---|
| 1 | L-asparagine | 3-4 gr |
| 2 | Monopotassium phosphate | 2-3 gr |
| 3 | Magnesium sulfate | 0,2-0,3 gr |
| 4 | Peptone | 15-20 gr |
| 5 | Agar | 15 gr |
| 6 | Magnesium citrate | 0,3-1 gr |
| 7 | Glycerol | 3-7 ml |
| 8 | Distilled water | 900-950 ml |

For the preparation of the solid medium, the substances of the above table are used, and the final solid medium product is obtained by the following culturing method, which is comprised by the process steps of:
- Mixing 3-4 grams of L-asparagine, 2-3 grams of monopotassium phosphate, 0.2-0.3 grams of magnesium sulfate, 15-20 grams of peptone, 15 grams of agar, 0.3-1 grams of magnesium citrate, 3-7 milliliters of glycerol in 900-950 milliliters of distilled water,
- Sterilizing of the mixture by autoclave for 15 minutes at atmospheric pressure of 1 at 121°C,
- Cooling of the mixture sterilized by autoclave for 15 minutes under atmospheric pressure of 1 at 121°C to 50°C,
- Adding 5-10% (50-100 milliliters) of filter-sterilized inactivated sheep serum and/or sheep plasma to the mixture cooled to 50°C,
- Developing the media by stirring the mixture to which filter-sterilized inactivated sheep serum and/or sheep plasma is added,
- Dispersion of the resulting medium into sterile tubes of 5-7 milliliters,
- Solidification of the medium dispersed in sterile tubes in a horizontal position.

The prepared solid medium is stored at +4°C until it is used in order to be prevent from the contamination and to be protected from moisture and light.

The prepared solid medium can be used in growing of mycobacteria from clinical samples. Analogously, it can also be used in susceptibility tests by adding anti-tuberculosis drugs in appropriate concentrations to the medium.

In addition to the solid medium described above, it is also possible to obtain medium in liquid form. The liquid medium and the solid medium vary in content and agar is not used in the liquid medium.

The prepared liquid medium contains L-asparagine, monopotassium phosphate, magnesium sulfate, peptone, magnesium citrate, glycerol and distilled water.

The compositions and the amounts of the medium obtained are shown in Table 2.

**Table 2: Table showing the compositions and the substance amounts of the medium**

| | Sequence No. | Compositions | Amount of substances |
|---|---|---|---|
| 1 | | L-asparagine | 3-4 gr |
| 2 | | Monopotassium phosphate | 2-3 gr |
| 3 | | Magnesium sulfate | 0,2-0,3 gr |
| 4 | | Peptone | 15-20 gr |
| 5 | | Magnesium citrate | 0,3-1 gr |
| 6 | | Glycerol | 3-7 ml |
| 7 | | Distilled water | 900-950 ml |

For the preparation of the liquid medium, the substances described in the above table are used and the final product that is liquid product is obtained by the following culturing method, which is comprised of the process steps of:
- Mixing 3-4 grams of L-asparagine, 2-3 grams of monopotassium phosphate, 0.2-0.3 grams of magnesium sulfate, 15-20 grams of peptone, 0.3-1 grams of magnesium citrate, 3-7 milliliters of glycerol in 900-950 milliliters of distilled water,
- Sterilizing of the mixture by autoclave for 15 minutes at atmospheric pressure of 1 at 121°C,
- Cooling of the mixture sterilized by autoclave for 15 minutes under atmospheric pressure of 1 at 121°C to 50°C,
- Adding 5-10% (50-100 milliliters) of filter-sterilized inactivated sheep serum and/or sheep plasma to the mixture cooled to 50°C,
- Developing the media by stirring the mixture to which filter-sterilized inactivated sheep serum and/or sheep plasma is added,
- Dispersion of the resulting medium into sterile tubes of 5-7 milliliters,

The prepared liquid medium is stored at +4°C until it is used. The solid and liquid medium that is subject matter of the invention provides the basic ingredients necessary for the growth of mycobacteria during the culturing stage and allows for the growth of the bacteria in a shorter time by the addition of inactivated sheep serum and/or sheep plasma as the enrichment.

Liquid medium can also be used in existing automated systems thereby minimizing the costs and the growth time.

Solid medium and liquid medium are both used in the growth of mycobacteria as well as in antibiotic susceptibility tests, wherein they have formulations that is readily applicable for enabling the growth of mycobacteria. By this means, cost and growth time of bacteria are decreased for the diagnosis to be conducted and for their antibiotic susceptibilities to be tested.

## Claims

1. A medium which increases growth rate of mycobacteria by using an enrichment of animal origin together with substances in the medium, shortens determination time of mycobacteria and used for antibiotic susceptibility tests of *M. tuberculosis,* **characterized in that**; a liquid form of the medium comprises 3-4 grams of L-asparagine, 2-3 grams of monopotassium phosphate, 0.2-0.3 grams of magnesium sulfate, 15-20 grams of peptone, 0.3-1 grams of magnesium citrate, 3-7 milliliters of glycerol, 900-950 milliliters of distilled water and inactive sheep serum and/or sheep plasma in the rate of 5-10% of final volume of resulting medium, and a solid form of agar medium comprises 15 grams of agar in addition to above mentioned substances.

2. A preparation method of the liquid medium form according to claim 1 which enhances the growth rate of mycobacteria by use of enrichment of animal origin together with substances in the medium and used for conducting antibiotic susceptibility tests of *M. tuberculosis* isolates, **characterized by** comprising the following process steps;
• Preparation of the medium mixture by mixing 3-4 grams of L-asparagine, 2-3 grams of monopotassium phosphate, 0.2-0.3 grams of magnesium sulfate, 15-20 grams of peptone, 0.3-1 grams of magnesium citrate, 3-7 milliliters of glycerol and 900-950 milliliters of distilled water,
• Sterilizing of the mixture by autoclave for 15 minutes at atmospheric pressure of 1 at 121°C,
• Cooling of the mixture sterilized by autoclave for 15 minutes under atmospheric pressure of 1 at 121°C to 50°C,
• Forming of the medium by adding 5-10% (50-100 milliliters) of filter-sterilized inactivated sheep serum and/or sheep plasma to the mixture cooled to 50°C,
• Dispersing 5-7 milliliters of the resulting medium into sterile tubes.

3. A preparation method of the solid agar medium form according to claim 1 in which the growth rate of mycobacteria is enhanced by use of enrichment of animal origin together with substances in the medium, and which is used for conducting antibiotic susceptibility tests of *M. tuberculosis* isolates, **characterized by** comprising the following process steps;
• Mixing of 3-4 grams of L-asparagine, 2-3 grams of monopotassium phosphate, 0.2-0.3 grams of magnesium sulfate, 15-20 grams of peptone, 0.3-1 grams of magnesium citrate, 3-7 milliliters of glycerol, 15 grams of agar and 900-950 milliliters of distilled water,
• Sterilizing of the mixture by autoclave for 15 minutes at atmospheric pressure of 1 at 121°C,
• Cooling of the mixture sterilized by autoclave for 15 minutes under atmospheric pressure of 1 at 121°C to 50°C,
• Forming of the medium by adding 5-10% (50-100 milliliters) of filter-sterilized inactivated sheep serum and/or sheep plasma to the mixture cooled to 50°C,
• Dispersing 5-7 milliliters of the resulting medium into sterile tubes.

## Patentansprüche

1. Medium, das die Wachstumsrate von Mykobakterien durch die Verwendung einer Anreicherung tierischen Ursprungs zusammen mit Substanzen in dem Medium erhöht, die Bestimmungszeit von Mykobakterien verkürzt und für Antibiotika-Sensibilitätstests von M. tuberculosis verwendet wird, **dadurch gekennzeichnet, dass** eine flüssige Form des Mediums 3-4 Gramm L-Asparagin, 2-3 Gramm Kaliumdihydrogenphosphat, 0.2-0.3 Gramm Magensiumsulfat, 15-20 Gramm Pepton, 0.3-1 Gramm Magnesiumhydrogencitrat, 3-7 Mililiter Glycerin, 900-950 Mililiter destilliertes Wasser und inaktives Schafserum und/oder Schafsplasma in Höhe von 5-10% des Endvolumens des resultierenden Mediums enthält, und eine feste Form von Agar-Nährmedium zusätzlich zu den oben erwähnten Substanzen 15 Gramm enthält.

2. Ein Zubereitungsverfahren der flüssigen Mediumform nach Anspruch 1, das die Wachstumsrate von Mykobakterien durch die Verwendung von tierischen Ursprungs zusammen mit Substanzen in dem Medium verbessert und für Durchführung von Antibiotika-Sensibilitatstests von M. tuberculosis-Isolaten, **dadurch gekennzeichnet, dass** es die folgenden Verfahrensschritte umfasst;
• Zubereitung der Mediummischung durch Mischen von 3-4 Gramm L-Asparagin, 2-3 Gramm Kaliumdihydrogenphosphat, 0.2-0.3 Gramm Magensiumsulfat, 15-20 Gramm Pepton, 0.3-1 Gramm Magnesiumhydrogencitrat, 3-7 Mililiter Glycerin, 900-950 Mililiter destilliertes Wasser,
• Sterilisieren des Gemischs im Autoklaven für 15 Minuten bei Atmosphärendruck 1 und 121°C,
• Abkühlung der von Autoklav sterilisierten Mischung für 15 Minuten unter dem Atmosphärendruck von 1 bei 121°C auf 50°C.
• Bildung des Mediums durch Hinzufügen von 5-10% (50-100 ml) filtersterilisiertem und inaktiviertem Schafserum und/oder Schafsplasma zu der auf 50°C gekühlten Mischung,
• Dispergierung von 5-7 Mililiter des resultierenden Mediums in sterile Röhrchen.

3. Ein Zubereitungsverfahren der festen Form von Agar-Nährmedium nach Anspruch 1, bei dem die Wachstumsrate von Mykobakterien durch die Verwendung der Anreicherung tierischen Ursprungs zusammen mit Substanzen in dem Medium verbessert wird und das für Durchführung von Antibiotika-Sensibilitatstests von M. tuberculosis-Isolaten, **dadurch gekennzeichnet, dass** es die folgenden Verfahrensschritte umfasst;
• Mischung von 3-4 Gramm L-Asparagin, 2-3 Gramm Kaliumdihydrogenphosphat, 0.2-0.3 Gramm Magensiumsulfat, 15-20 Gramm Pepton, 0.3-1 Gramm Magnesiumhydrogencitrat, 3-7 Mililiter Glycerin, 900-950 Mililiter destilliertes Wasser,
• Sterilisieren des Gemischs im Autoklaven für 15 Minuten bei Atmosphärendruck 1 und 121°C,
• Abkühlung der von Autoklav sterilisierten Mischung für 15 Minuten unter dem Atmosphärendruck von 1 bei 121°C auf 50°C.
• Bildung des Mediums durch Hinzufügen von 5-10% (50-100 ml) filtersterilisiertem und inaktiviertem Schafserum und/oder Schafsplasma zu der auf 50°C gekühlten Mischung,
• Dispergierung von 5-7 Mililiter des resultierenden Mediums in sterile Röhrchen.

## Revendications

1. Milieu qui augmente le taux de croissance des mycobactéries en utilisant un enrichissement d'origine animale en même temps que des substances dans le milieu, qui raccourcit le temps de détermination des mycobactéries et qui est utilisé pour les tests de sensibilité aux antibiotiques de M. tuberculosis, **caractérisé en ce que**; une forme liquide du milieu comprend 3-4 grammes de L-asparagine, 2-3 grammes de phosphate monopotassique, 0.2-0.3 grammes de sulfate de magnésium, 15-20 grammes de peptone, 0.3-1 gramme de citrate de magnésium, 3-7 millilitres de glycérol, 900-950 millilitres d'eau distillée et du sérum de mouton inactif et/ou du plasma de mouton à raison de 5-10% du volume final du milieu résultant, et une forme solide de milieu agar comprend 15 grammes d'agar en plus des substances mentionnées ci-dessus.

2. Procédé de préparation de la forme de milieu liquide selon la revendication 1 qui
améliore le taux de croissance des mycobactéries
par l'utilisation d'un enrichissement d'origine animale avec des substances dans le milieu et utilisé pour réaliser des tests de sensibilité aux antibiotiques d'isolats de M. tuberculosis, **caractérisé en ce qu'**il comprend les étapes de procédé suivantes;
• Préparation du mélange de milieu en mélangeant 3-4 grammes de L-asparagine, 2-3 grammes de phosphate monopotassique, 0.2-0.3 grammes de sulfate de magnésium, 15-20 grammes de peptone, 0.3-1 grammes de citrate de magnésium, 3-7 millilitres de glycérol et 900-950 millilitres d'eau distillée,
• Stérilisation du mélange par autoclave pendant 15 minutes à la pression atmosphérique de 1 à 121°C,
• Refroidissement du mélange stérilisé par autoclave pendant 15 minutes sous la pression atmosphérique de 1 à 121°C à 50°C,
• Formation du milieu par l'addition de 5-10% (50-100 millilitres) de sérum de mouton et/ou de plasma de mouton inactivé stérilisé par filtration au mélange refroidi à 50°C,
• Dispersion de 5-7 millilitres du milieu obtenu dans des tubes stériles.

3. Procédé de préparation de la forme de milieu agar solide selon la revendication 1, dans lequel le taux de croissance des mycobactéries est amélioré par l'utilisation d'un enrichissement d'origine animale conjointement avec des substances dans le milieu, et qui est utilisé pour réaliser des tests de sensibilité aux antibiotiques d'isolats de M. tuberculosis, **caractérisé en ce qu'**il comprend les étapes de procédé suivantes ;
• Mélange de 3-4 grammes de L-asparagine, 2-3 grammes de phosphate monopotassique, 0.2-0.3 grammes de sulfate de magnésium, 15-20 grammes de peptone, 0.3-1 grammes de citrate de magnésium, 3-7 millilitres de glycérol, 15 grammes d'agar et 900-950 millilitres d'eau distillée,
• Stérilisation du mélange par autoclave pendant 15 minutes à la pression atmosphérique de 1 à 121°C,
• Refroidissement du mélange stérilisé par autoclave pendant 15 minutes sous pression atmosphérique de 1 à 121°C à 50°C,
• Formation du milieu par addition de 5-10% (50-100 millilitres) de sérum de mouton et/ou de plasma de mouton inactivé stérilisé par filtration au mélange refroidi à 50°C,
• Dispersion de 5-7 millilitres du milieu obtenu dans des tubes stériles.
